# EUROPEAN PATENT APPLICATION

(11) **EP 4 470 601 A1**
(43) Date of publication of application: **04.12.2024**
(21) Application number: 23746479.7
(22) Date of filing: 30.01.2023
(51) Int. Cl.: A61N 1/365

(54) **PULSE STIMULATION CONTROL METHOD AND APPARATUS, MEDICAL SYSTEM, ELECTRONIC DEVICE, AND MEDIUM**

(30) Priority: 30.01.2022 CN 202210114130
(71) Applicant: United Innomed (Shanghai) Limited, Shanghai 201315 (CN)
(72) Inventor: WANG, Li, Shanghai 201315 (CN)
(74) Representative: dompatent von Kreisler Selting Werner - Partnerschaft von Patent- und Rechtsanwälten mbB
(86) International application number: PCT/CN2023/073806
(87) International publication number: WO 2023/143602

(57) **Abstract**

Disclosed are a pulse stimulation control method and apparatus, a medical system, an electronic device, and a medium. The method comprises: acquiring a first sensing time of an R wave in a preset electrocardiogram; determining a sensing event and a second sensing time thereof in a local myocardial electrocardiogram corresponding to a set myocardial position; and when the second sensing time does not meet a first preset condition, determining that the sensing event is not an R wave signal corresponding to the R wave in the preset electrocardiogram, and controlling not to emit corresponding pulse simulation. According to the present invention, it is ensured that in a wrong situation, the pulse stimulation on the heart is not emitted, thereby effectively avoiding the risk of inducing VT or VF. It is ensured that only when the R wave is determined, the pulse stimulation is emitted, and the pulse stimulation is emitted during an emitting period corresponding to the R wave in the preset electrocardiogram, so that the reliability, safety, timeliness, and effectiveness of the pulse stimulation on the heart of a patient are ensured, thereby better guaranteeing the safety, effectiveness, and therapeutic effect of the pulse stimulation on the patient.

## Description

The present application claims the priority of Chinese Patent Application No. 2022101141302 filed on January 30, 2022, and the content of which is incorporated herein by reference in its entirety.

### TECHNICAL FIELD

The present disclosure relates to the technical field of medical devices, and in particular, to a pulse stimulation control method and apparatus, a medical system, an electronic device, and a medium.

### BACKGROUND

The existing medical apparatus with Cardiac Contractility Modulation (CCM) functionality in the market is primarily used for patients with chronic heart failure. It typically involves the implantation of two bipolar leads into the right ventricular septum to sense local myocardial potentials and deliver pulse stimulation at a certain time after the sensing to enhance ventricular myocardial contractility. The two bipolar electrodes are used to sense the electrical activity of the local myocardium and the timing sequence of the electrical activity. Both electrodes can be used to trigger pulse stimulation (herein also called CCM stimulation) through the local activation of myocardium within the deliverable period of the local myocardium (also referred to as the safety period, corresponding to the absolute refractory period of the local myocardium). Pulse stimulation is only delivered during ventricular beats (electrical activity) conducted from the atrium and not during premature ventricular contractions (PVC) or beats initiated from the ventricle.

Pulse stimulation is delivered within the deliverable period of the local myocardium, thereby ensuring effectiveness while avoiding re-depolarization of the local myocardium. However, the energy of pulse stimulation is quite significant. Such high-energy electrical stimulation may affect far-field myocardium beyond the local myocardium in contact with the stimulation electrode. Moreover, the extent of this impact varies with the design (e.g., shape) of the stimulation electrodes at the local myocardium and the distance between electrodes. Under the same energy, a greater distance between stimulation electrodes or a larger effective area of the stimulation electrodes may result in a greater potential impact and/or range on far-field myocardium. If the local pulse stimulation occurs at a site where the myocardial repolarization (within the absolute refractory period) is late, there is a possibility that the pulse stimulation is delivered after the deliverable period of action potentials of the far-field myocardium, which has undergone earlier depolarization (relative to the local myocardium). This could increase the likelihood of this part of the myocardium experiencing re-depolarization, thereby potentially triggering malignant ventricular arrhythmias (VT/VF, ventricular tachycardia or ventricular fibrillation). Existing pulse stimulation schemes involve delivering pulses on the ventricular septum. The atrial conduction activates this area of ventricular muscle earlier than other areas of the ventricular muscle, such as the left ventricular lateral wall. In this way, the pulses delivered on the ventricular septum do not fall after the deliverable period of other areas, thereby reducing or avoiding the risk of triggering ventricular arrhythmias in the myocardium of these areas. However, if ventricular excitation is not due to atrial-conducted electrical activity but rather due to intrinsic ventricular electrical activity, such as premature beats or ventricular pacing, the electrical activity of ventricular muscle on the ventricular septum may not necessarily be the earliest in the ventricular muscle. In this case, pulse stimulation delivered on the ventricular septum may fall after the deliverable period of myocardium in other areas, thus increasing the risk of ventricular arrhythmias being induced in those myocardial areas by pulsed electrical stimulation. Therefore, existing pulse stimulation apparatuses do not deliver pulse stimulation during ventricular excitations not caused by the atrium (ventricular ectopic excitations). However, the occurrence rate of premature ventricular contractions in heart failure patients is high. Some heart failure patients rely on ventricular pacing for long periods, including patients with reduced heart rates due to long-term beta-blocker use, patients with single-chamber ICDs, and patients with cardiac resynchronization therapy (CRT). The opportunity of delivering pulse stimulation on myocardium for these patients may be significantly reduced as a result. For myocardial stimulation electrodes placed in other sites of the ventricle (e.g., sites outside the ventricular septum), the likelihood of occurring similar risks described above may be higher.

In the existing pulse stimulation scheme, upon detecting a sensing event in the local myocardial electrocardiogram of a certain myocardial position, pulse stimulation can be directly delivered to the stimulation electrode at that myocardial position after a certain period of time. However, if a sensing event occurs erroneously in the local myocardial electrocardiogram of a certain myocardial position, which means the sensing event is a T-wave or another event (such as interference signals), and pulse stimulation still occurs after the T-wave. This significantly increases the risk of inducing malignant ventricular arrhythmias such as VT or VF, thereby increasing the patient's risk of mortality and subjecting the patient to additional painful stimulation.

### CONTENT OF THE PRESENT INVENTION

The present disclosure is intended to overcome the defects in the prior art where the pulse stimulation scheme is directly based on the presence of a sensing event in the local myocardial electrocardiogram of a certain myocardial position to determine the delivery of pulse stimulation to the corresponding myocardium, which may lead to the risk of inducing malignant ventricular arrhythmias such as VT or VF during false sensing events, and pulse stimulation is only delivered to the atrial septum during atrial-conducted ventricular electrical activity but cannot be delivered during ventricular electrical activity caused by the ventricle itself. The objective of the present disclosure is to provide a pulse stimulation control method and apparatus, a medical system, an electronic device, and a medium. Pulse stimulation electrodes may be placed at different positions (such as the left ventricular wall) or a plurality of positions of the ventricle, which aims to better enhance the contractility of the heart while ensuring that the risk of inducing ventricular arrhythmias is minimized during this process, thereby effectively ensuring the safety and therapeutic effect of pulse stimulation for patients.

The present disclosure solves the above technical problems through the following technical solutions:
The present disclosure provides a pulse stimulation control method, comprising:
acquiring a first sensing time of an R-wave in a preset electrocardiogram;
determining a sensing event in a local myocardial electrocardiogram corresponding to a set myocardial position based on the first sensing time, and acquiring a second sensing time corresponding to the sensing event; and
determining that the sensing event is not an R-wave signal corresponding to the R-wave in the preset electrocardiogram if the second sensing time does not meet a first preset condition, and then not delivering pulse stimulation to a stimulation electrode at the set myocardial position,
wherein the first preset condition is determined based on the first sensing time of the R-wave in the preset electrocardiogram and the second sensing time corresponding to the sensing event.

Preferably, determining that the sensing event is not the R-wave signal corresponding to the R-wave in the preset electrocardiogram if the second sensing time does not meet the first preset condition, and then not delivering the pulse stimulation to the stimulation electrode at the set myocardial position, comprises:
acquiring a sensing time window corresponding to the R-wave in the preset electrocardiogram; and
determining that the sensing event is not the R-wave signal corresponding to the R-wave in the preset electrocardiogram if the second sensing time does not fall within the sensing time window, and then not delivering the pulse stimulation to the stimulation electrode at the set myocardial position.

Preferably, determining the sensing event in the local myocardial electrocardiogram corresponding to the set myocardial position based on the first sensing time, comprises:
taking the first sensing time as a first time point, and taking a time point from corresponding first set duration before the first time point as a time reference starting point; and
acquiring the sensing event in the local myocardial electrocardiogram corresponding to the set myocardial position based on the time reference starting point.

Preferably, the method further comprises:
determining the sensing event as an R-wave if the second sensing time falls within the sensing time window.

Preferably, the pulse stimulation control method further comprises:
determining, after the sensing event is determined as the R-wave, a pulse delivery time corresponding to the stimulation electrode at the set myocardial position based on the second sensing time of the R-wave in the local myocardial electrocardiogram; and
determining whether the pulse delivery time meets a second preset condition, and if yes, determining to deliver the pulse stimulation to the stimulation electrode at the set myocardial position; otherwise, not delivering the pulse stimulation to the stimulation electrode at the set myocardial position.

Preferably, determining whether the pulse delivery time meets the second preset condition, and if yes, determining to deliver the pulse stimulation to the stimulation electrode at the set myocardial position; otherwise, not delivering the pulse stimulation to the stimulation electrode at the set myocardial position, comprises:
acquiring a deliverable pulse time window corresponding to the R-wave in the preset electrocardiogram,
wherein the deliverable pulse time window is set to correspond with the sensing time window;
controlling the pulse stimulation to be delivered to the stimulation electrode at the set myocardial position in the pulse delivery time if the pulse delivery time falls within the deliverable pulse time window; and
not delivering the pulse stimulation to the stimulation electrode at the set myocardial position if the pulse delivery time does not fall within the deliverable pulse time window.

Preferably, when there are a plurality of the set myocardial positions, the method further comprises:
determining whether the corresponding second sensing time falls within the sensing time window for each of the second sensing times of the sensing events occurring in each of the local myocardial electrocardiograms in sequence,
wherein the sensing time window is determined based on the first sensing time of the R-wave in the preset electrocardiogram and the second sensing time corresponding to the sensing event that occurs first.

Preferably, when there are a plurality of the set myocardial positions, the method further comprises:
presetting set sensing parameters corresponding to sensing times at different set myocardial positions,
wherein the set sensing parameter comprises a set sensing time and/or a set sensing occurrence sequence; or,
setting sensing events in the other remaining local myocardial electrocardiograms all as R-waves if the sensing event in the local myocardial electrocardiogram that occurs first is an R-wave; or,
setting the sensing events in the other remaining local myocardial electrocardiograms all as R-waves if the sensing event in the local myocardial electrocardiogram that occurs last is an R-wave.

Preferably, the preset electrocardiogram comprises a surface electrocardiogram and/or an *in vivo* far-field myocardial electrocardiogram;
and/or,
the deliverable pulse time window and the sensing time window are determined based on the preset electrocardiogram.

Preferably, determining the pulse delivery time corresponding to the stimulation electrode at the set myocardial position based on the second sensing time of the R-wave in the local myocardial electrocardiogram, comprises:
calculating the pulse delivery time corresponding to the stimulation electrode at the set myocardial position based on the second sensing time of the R-wave in the local myocardial electrocardiogram and a preset duration;
   or,
calculating a time difference between the first sensing time corresponding to the R-wave in the preset electrocardiogram and the second sensing time of the set myocardial position; and
using the first sensing time as a reference zero point, calculating the pulse delivery time corresponding to the set myocardial position based on the time difference and the preset duration.

Preferably, the pulse stimulation is CCM stimulation,
wherein the CCM stimulation may be delivered during at least one of the following ventricular electrical activities:
sinus beats, ventricular beats resulting from atrial conduction, ventricular beats originating from ventricles, and ventricular paced beats.

The present disclosure further provides a pulse stimulation control apparatus, comprising:
a first sensing time acquisition module, configured to acquire a first sensing time of an R-wave in a preset electrocardiogram;
a sensing event determination module, configured to determine a sensing event in a local myocardial electrocardiogram corresponding to a set myocardial position based on the first sensing time;
a second sensing time acquisition module, configured to acquire a second sensing time corresponding to the sensing event; and
a first judgment module, configured to determine that the sensing event is not an R-wave signal corresponding to the R-wave in the preset electrocardiogram if the second sensing time does not meet a first preset condition, and then not deliver pulse stimulation to a stimulation electrode at the set myocardial position,
wherein the first preset condition is determined based on the first sensing time of the R-wave in the preset electrocardiogram and the second sensing time corresponding to the sensing event.

The present disclosure further provides a medical system, comprising the aforementioned pulse stimulation control apparatus.

The present disclosure further provides an electronic device, comprising a memory, a processor, and a computer program stored on the memory and executable by the processor, wherein the processor, when executing the computer program, implements the aforementioned pulse stimulation control method.

The present disclosure further provides a computer-readable storage medium storing a computer program, wherein the computer program, when executed by a processor, causes the processor to implement the aforementioned pulse stimulation control method.

On the basis of the general knowledge in the art, the preferred conditions can be combined arbitrarily to obtain preferred embodiments of the present disclosure.

The positive and progressive effects of the present disclosure are as follows:
(1) Pulse stimulation is delivered only after the R-wave: In the present disclosure, it is possible to timely analyze and process the sensing event in the local myocardial electrocardiogram of the myocardium. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively reducing or avoiding the risk of inducing VT or VF, thereby preventing unnecessary pain or even safety hazards to patients. This ensures patient safety and enhances the reliability of pulse stimulation control; additionally, it ensures that pulse stimulation is delivered timely when the sensing event is confirmed as an R-wave, that is, pulse stimulation is delivered only under correct conditions.
(2) Pulse stimulation is only delivered during the ventricular deliverable period: By confirming that the pulse stimulation is delivered within the deliverable period corresponding to the overall ventricular electrical activity (R-wave) reflected in the surface electrocardiogram or the *in vivo* far-field myocardial electrocardiogram, the timeliness, safety, and effectiveness of pulse stimulation to the patient's heart are guaranteed. Where to ensure the safety of the pulse stimulation transmission time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of pulse stimulation for the patient.

### BRIEF DESCRIPTION OF THE DRAWING

FIG. 1 is a flowchart of a pulse stimulation control method according to Embodiment 1 of the present disclosure.
FIG. 2 is the first flowchart of a pulse stimulation control method according to Embodiment 2 of the present disclosure.
FIG. 3 is the second flowchart of the pulse stimulation control method according to Embodiment 2 of the present disclosure.
FIG. 4 is the third flowchart of the pulse stimulation control method according to Embodiment 2 of the present disclosure.
FIG. 5 is the fourth flowchart of the pulse stimulation control method according to Embodiment 2 of the present disclosure.
FIG. 6 is the fifth flowchart of the pulse stimulation control method according to Embodiment 2 of the present disclosure.
FIG. 7 is a schematic electrocardiogram corresponding to R-wave sensing in the case of a single electrode lead according to Embodiment 2 of the present disclosure.
FIG. 8 is a schematic electrocardiogram corresponding to R-wave sensing in the case of a plurality of electrode leads according to Embodiment 2 of the present disclosure.
FIG. 9 is a schematic diagram illustrating modules of a pulse stimulation control system according to Embodiment 3 of the present disclosure.
FIG. 10 is a schematic diagram illustrating modules of a pulse stimulation control system according to Embodiment 4 of the present disclosure.
FIG. 11 is a structural schematic diagram of an electronic device for implementing the pulse stimulation control method according to Embodiment 5 of the present disclosure.

### DETAILED DESCRIPTION OF THE PREFERRED EMBODIMENT

The following further illustrates the present disclosure through embodiments, but the present disclosure is not limited thereby to the scope of the disclosed embodiments.

In the present disclosure, stimulation electrodes are pre-set at different myocardial positions of the patient (the myocardial position for setting the electrodes may be determined or adjusted according to actual needs). Each stimulation electrode is electrically connected to implanted and/or external pulse stimulation devices via leads to achieve control operations such as collecting cardiac electrical signals from different myocardial positions and delivering pulse stimulation to different myocardial positions.

The pulse stimulation scheme of the present disclosure may be used for situations involving a single electrode lead for pulse stimulation, as well as situations involving multiple electrode leads for pulse stimulation, such as the Electrical Circulatory Support (ECS) system, which provides pulse stimulation to multiple myocardial sites, thus covering one or more cardiac chambers most in need of local and overall support; the system can better ensure the timeliness, safety, and effectiveness of pulse stimulation provided by the system while providing the pulse stimulation.

### Embodiment 1

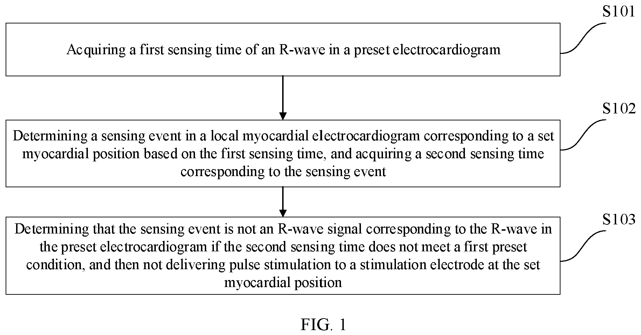

As shown in FIG. 1, the pulse stimulation control method according to this embodiment includes:
S101, acquiring the first sensing time of the R-wave in a preset electrocardiogram,
wherein the preset electrocardiogram includes but is not limited to the surface electrocardiogram (ECG) and the *in vivo* far-field myocardial electrocardiogram (Far-Field Electrogram, also referred to as FF-EGM); the first sensing time is also referred to as global sense (GS, far-field sensing).
S102, determining the sensing event in the local myocardial electrocardiogram corresponding to the set myocardial position based on the first sensing time, and acquiring the second sensing time corresponding to the sensing event,
wherein the second sensing time corresponding to the sensing event in the local myocardial electrocardiogram is also referred to as local sense (LS, near-field sensing); and
the sensing event in the local myocardial electrocardiogram is a sensing event corresponding to the R-wave in the preset electrocardiogram; and
S103, determining that the sensing event is not the R-wave signal corresponding to the R-wave in the preset electrocardiogram if the second sensing time does not meet the first preset condition, and then not delivering pulse stimulation to the stimulation electrode at the set myocardial position,
wherein the first preset condition is determined based on the first sensing time of the R-wave in the preset electrocardiogram and the second sensing time corresponding to the sensing event.

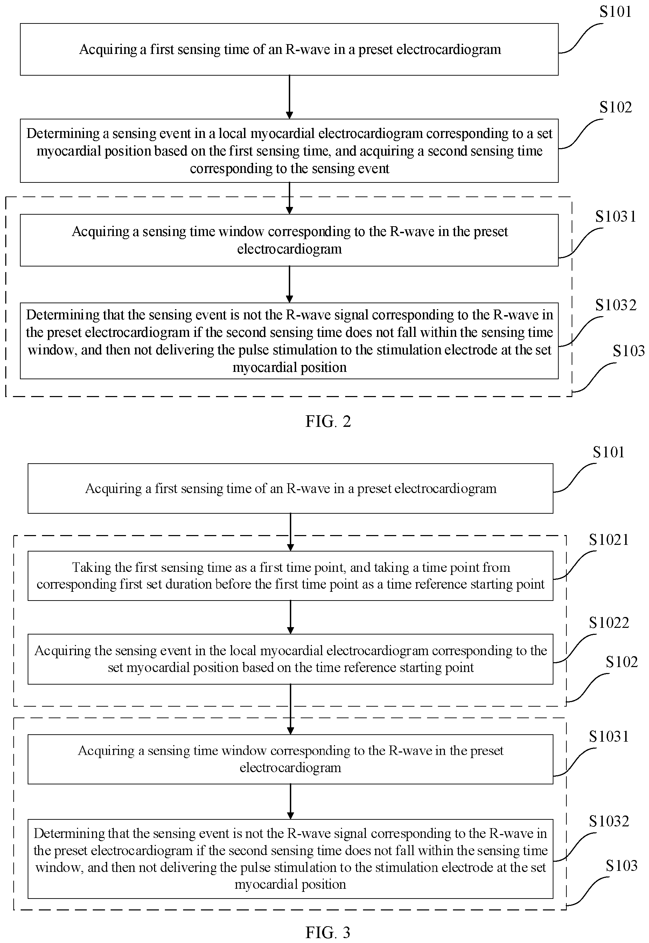

In one implementation, as shown in FIG. 2, step S103 includes:
S1031, acquiring the sensing time window corresponding to the R-wave in the preset electrocardiogram,
wherein the method for acquiring the sensing time window includes but is not limited to: obtaining the sensing time window based on a preset sensing circuit or directly receiving the sensing time window through an external device input.

Surface electrocardiogram (ECG): The corresponding signal is derived from control electrodes attached to the skin; the control electrodes herein include but are not limited to commonly used surface ECG electrodes and specially designed electrodes.

The far-field myocardial electrocardiogram (FF-EGM) represents (or reflects) the signals of the overall cardiac electrical activity, similar to the surface electrocardiogram. The signals corresponding to the far-field myocardial electrocardiogram come from various combinations of control electrodes, including but not limited to electrodes directly contacting the myocardium with a relatively large surface area (i.e., significantly larger than the surface area of conventional electrodes used for electrical stimulation of the myocardium), electrodes not in direct contact with the myocardium but positioned near or distant from the ventricular myocardium, or electrodes with relatively large surface areas located subcutaneously.

For example, different combinations of electrodes correspond to: any combination of different electrodes such as those placed in blood vessels or cardiac chambers, defibrillation electrodes placed on the epicardium, subcutaneous defibrillation electrodes for sub-Q ICDs, or defibrillation electrodes for external defibrillators (such as AEDs). The specific arrangement of these different electrodes may be determined or adjusted based on the requirements of the actual usage scenario.

The sensing time and the corresponding sensing time window may be acquired based on an R-wave in a surface electrocardiogram via a single lead or multiple leads, and/or an R-wave or R-waves in one or more far-field myocardial electrocardiograms. They represent part or all of the time period of overall ventricular myocardial depolarization electrical activity, thereby obtaining the deliverable pulse time window. By improving the control mechanism for determining whether to deliver stimulation at the stimulation site and the timing of delivery, the accuracy of determining pulse stimulation delivery is further enhanced, thereby increasing its safety.

Step S103 further includes S1032: determining that the sensing event is not the R-wave signal corresponding to the R-wave in the preset electrocardiogram if the second sensing time does not fall within the sensing time window, and then not delivering pulse stimulation to the stimulation electrode at the set myocardial position.

Where non-R-wave signals corresponding to the sensing event LS in the local myocardial electrocardiogram include T-wave signals or other interference signals. Specifically, pulse stimulation is CCM stimulation; allowing pulse stimulation to be implemented under different ventricular electrical activities expands the applicability of pulse stimulation, significantly enhancing the overall effectiveness of pulse stimulation control.

CCM stimulation occurs during the following ventricular electrical activities: sinus beats, ventricular beats conducted from atrium, ventricular beats originating from the ventricles (ventricular ectopic excitations), ventricular paced beats, etc.

Confirming that the local sensing event is an R-wave better ensures that CCM stimulation is triggered by the local R-wave instead of other signals (such as T-waves, myoelectricity, or other non-myocardial depolarization electrical activities), effectively eliminating the occurrence of false triggers. Through the design of this critical step, the safety and effectiveness of pulse stimulation can be effectively improved.

To effectively improve the confirmation precision of whether LS in the local myocardial electrocardiogram is R-wave sensing, this embodiment simultaneously considers both far-field sensing (GS) and near-field sensing (LS) of the same heartbeat. By jointly determining the judgment result through both far-field sensing (GS) and near-field sensing (LS) under the same heartbeat, the probability of false triggering of pulse stimulation due to potential misjudgment when only considering one type of sensing (either GS or LS) is greatly reduced. Consequently, this significantly enhances the confirmation accuracy of the sensing event (LS) and further effectively prevents pulse stimulation from being delivered under incorrect conditions, greatly improving the safety of pulse stimulation for patients.

Ensuring that pulse stimulation is delivered not only during the deliverable period of the local myocardial R-wave (sensed by the electrode at this myocardial site) but also during the deliverable period of the far-field myocardial R-wave (electrical activity generated by the ventricular myocardium as a whole) minimizes and even eliminates the risk of ventricular myocardium depolarization due to inadvertent activation of the pulse stimulation.

In this embodiment, the sensing event in the local myocardial electrocardiogram for each heartbeat can be analyzed and processed timely. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, and preventing unnecessary pain or even safety hazards to patients. This ensures patient safety, enhances the reliability of pulse stimulation control, and guarantees the safety, effectiveness, and therapeutic effects of pulse stimulation for patients.

### Embodiment 2

The pulse stimulation control method according to this embodiment is a further improvement of Embodiment 1, specifically:
In one implementation, as shown in FIG. 3, step S102 includes:
S1021, taking the first sensing time as the first time point, and taking the time point from corresponding first set duration before the first time point as the time reference starting point; and
S1022, acquiring the sensing event in the local myocardial electrocardiogram corresponding to the set myocardial position based on the time reference starting point.

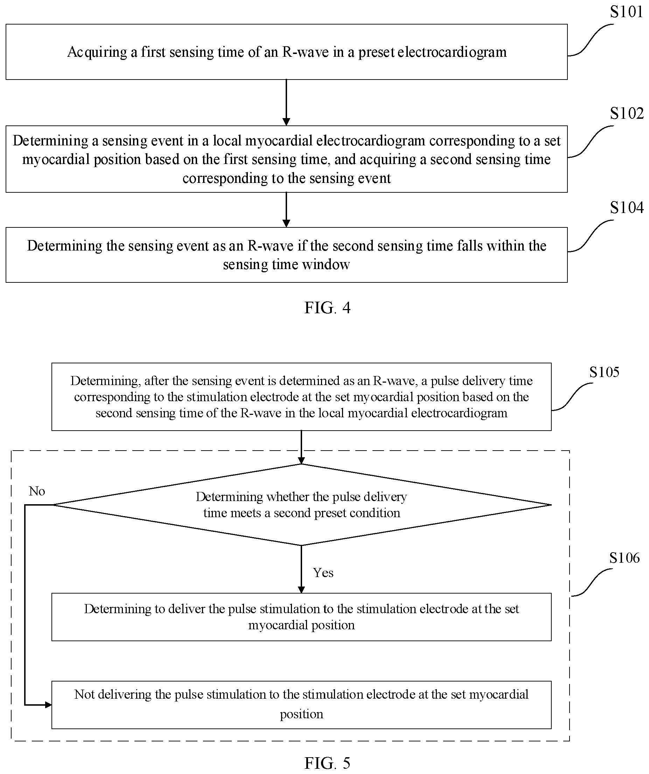

In one implementation, as shown in FIG. 4, step S102 is followed by:
S104, determining the sensing event as an R-wave if the second sensing time falls within the sensing time window.

In one implementation, as shown in FIG. 5, the pulse stimulation control method according to this embodiment further includes:
S105, determining, after the sensing event is determined as an R-wave, the pulse delivery time corresponding to the stimulation electrode at the set myocardial position based on the second sensing time of the R-wave in the local myocardial electrocardiogram.

It should be noted that in this embodiment, the method of acquiring sensing events as R-waves may be directly obtained through transmission by external devices, or determined based on judgments such as whether it falls within the sensing time window.

Further included is S106: determining whether the pulse delivery time meets the second preset condition, and if yes, determining to deliver pulse stimulation to the stimulation electrode at the set myocardial position; otherwise, not delivering pulse stimulation to the stimulation electrode at the set myocardial position.

The determination of the sensing event of which the local myocardial sensing time is within the sensing time window corresponding to the R-wave in the preset electrocardiogram ensures that the sensing event belongs to the R-wave signal of the local myocardium corresponding to the R-wave in the preset electrocardiogram. This guarantees the timely delivery of pulse stimulation only when the event is determined as an R-wave, ensuring the timeliness, safety, and effectiveness of pulse stimulation for the patient's heart.

Ensuring that pulse stimulation is delivered not only during the deliverable period of the local myocardial R-wave (sensed by the electrode at this myocardial site) but also during the deliverable period of the far-field myocardial R-wave (electrical activity generated by the ventricular myocardium as a whole) minimizes and even eliminates the risk of the depolarization of other sites of the ventricular myocardium due to the activation of the pulse stimulation. This significantly enhances the safety of pulse stimulation for patients, minimizing unnecessary treatment risks and pain.

In other words, the pulse stimulation control of this embodiment ensures that regardless of where the stimulation electrode is located in the myocardium, pulse stimulation is only delivered during the deliverable period of both the local myocardium and the entire ventricular myocardium under correct conditions. This better ensures the safety and effectiveness of pulse stimulation, allowing pulse stimulation to be delivered under various heartbeats as needed (including ventricular beats caused by atrial conduction, ventricular beats originating from the ventricles themselves, such as PVCs and ventricular paced beats), thereby better meeting the patient's demand for enhanced myocardial contractility.

In one implementation, step S 105 includes:
calculating the pulse delivery time corresponding to the stimulation electrode at the set myocardial position based on the second sensing time of the R-wave in the local myocardial electrocardiogram and the preset duration;
   or,
calculating the time difference between the first sensing time (GS) corresponding to the R-wave in the preset electrocardiogram and the second sensing time of the set myocardial position; and
using the first sensing time as the reference zero point, calculating the pulse delivery time corresponding to the set myocardial position based on the time difference and the preset duration.

The following are the detailed explanations of the processes for calculating the pulse delivery time of the R-wave in the local myocardial electrocardiogram according to the two methods mentioned above:
(1) The second sensing time when the R-wave appears in the local myocardial electrocardiogram is acquired timely, the second sensing time is used as the reference zero point (or called trigger point), and on this basis, the pulse delivery time corresponding to the stimulation electrode at the corresponding myocardial position is obtained by adding the preset duration (LPD).
(2) Based on the second sensing time and the first sensing time when the R-wave appears in the surface electrocardiogram (ECG) and/or the far-field myocardial electrocardiogram (FF-EGM), the time difference (GLSD) between the second sensing time and the first sensing time is calculated; then, using the first sensing time as the reference zero point, and on this basis, the pulse delivery time (GPD) corresponding to each set myocardial position with the first sensing time as the trigger point (reference point) is calculated by adding the time difference (GLSD) and the preset duration (LPD), that is, GPD = GLSD + LPD.

After obtaining the pulse delivery time (GPD) for each set myocardial position, the time difference may be maintained unchanged to deliver cardiac pulse stimulation to the corresponding control electrode based on the pulse delivery time (GPD); there is no need to recalculate before each electrical stimulation output, as the electrical stimulation output time may be directly determined based on GPD after the sensing of the R-wave in the local myocardial electrocardiogram. Directly determining the electrical stimulation output time based on GPD eliminates the need for calculating the pulse stimulation delivery time using the first and second sensing times each time, effectively reducing data processing time and improving the efficiency of controlling cardiac pulse stimulation triggers while achieving the myocardial stimulation effects.

In addition, the pulse delivery time (GPD) may be updated regularly or irregularly based on actual needs (where myocardial electrical stimulation may continue or stop). Then, myocardial electrical stimulation continues based on the updated trigger time to achieve more flexible electrical stimulation effects and meet the requirements of various pulse electrical stimulation scenarios.

For pulse stimulation triggered by the R-wave in the local myocardial electrocardiogram (second sensing time), the duration from the R-wave to delivery (LPD) is fixed for each stimulation position (such as a preset duration of 40 ms). For pulse stimulation triggered by the R-wave in the surface electrocardiogram (ECG) and/or far-field myocardial electrocardiogram (FF-EGM), the duration for each stimulation position (GPD) is variable and differs (i.e., determined by a fixed preset duration of 40 ms and a time difference that varies with position). Where the preset duration is typically defaulted to 40 ms, and the duration value may be fine-tuned or updated according to actual needs (i.e., programmable adjustment).

It needs to be noted that the GLSD and GPD corresponding to each set myocardial site can be measured and averaged over several intrinsic heartbeats during the set-up period (defaulting to five consecutive intrinsic heartbeats, ranging from 3 to 12). Alternatively, the GLSD and GPD can be obtained based on the measured duration under each heartbeat. The GPD of each set myocardial site or position is used to trigger the timing of pulse stimulation transmission at that site or position relative to the R-wave sensing moment in the preset electrocardiogram. The pulse delivery time corresponds to the duration from sensing the R-wave to triggering the delivery of cardiac pulse stimulation. The transmission of pulse stimulation at various positions or sites is triggered by the R-wave sensing of surface electrocardiogram (ECG) or the R-wave sensing of far-field myocardial electrocardiogram (FF-EGM), followed by delivering cardiac pulse stimulation to each corresponding control electrode based on the pulse delivery time. To ensure the effectiveness of pulse stimulation, all R-waves (i.e., R-waves in the far-field myocardial electrocardiogram FF-EGM and the surface electrocardiogram ECG) are sensed under the same heartbeat. Based on the aforementioned scheme for acquiring pulse delivery time, the timely and reliable delivery of pulse stimulation can be effectively ensured.

In addition, according to actual needs, GLSD and other parameters compatible with different ventricular electrical activities may be preset, for example, for sinus rhythm (SR) ventricular electrical activity. Then, during actual operation, the corresponding parameters set in the set-up period are directly called upon during the sinus rhythm, reducing the computational requirements for each heartbeat while ensuring the timeliness and effectiveness of pulse stimulation control.

Furthermore, GLSD and other parameters corresponding to the current actual ventricular electrical activity can be dynamically calculated based on real-time cardiac electrical activity sensing data and parameters during the operating period, without relying on parameters such as GLSD obtained during the set-up period. This further improves the timeliness and effectiveness of pulse stimulation control, thereby further ensuring the safety and efficacy of pulse stimulation for patients.

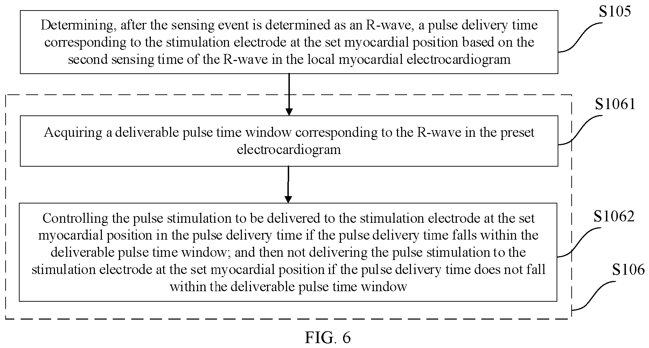

In one implementation, as shown in FIG. 6, step S106 includes:
S1061: acquiring the deliverable pulse time window corresponding to the R-wave in the preset electrocardiogram,
wherein the starting time point and the window duration corresponding to the deliverable pulse time window and the sensing time window can be the same or different; that is, the deliverable pulse time window and the sensing time window may be independently set without mutual correlation or dependency.

Specifically, the deliverable pulse time window and the sensing time window are determined based on the preset electrocardiogram, and the specific settings are adaptively adjusted based on factors such as the actual R-wave sensing and myocardial conditions, such that the requirements of the vast majority of pulse stimulation scenarios can be covered or met.

Step S106 further includes S1062: controlling the pulse stimulation to be delivered to the stimulation electrode at the set myocardial position in the pulse delivery time if the pulse delivery time falls within the deliverable pulse time window; and
not delivering the pulse stimulation to the stimulation electrode at the set myocardial position if the pulse delivery time does not fall within the deliverable pulse time window.

This scheme ensures that CCM stimulation must occur within the deliverable period of local myocardial depolarization (R-wave) and the deliverable period of *in vivo* far-field myocardial depolarization (R-wave). Where the deliverable pulse time window corresponds to a safe period, which corresponds to the deliverable period of the entire ventricle. Both the starting point and endpoint of the deliverable pulse time window are adjustable, with the goal of having the starting point approximately correspond to the depolarization zone and having the endpoint occur earlier than or equal to the endpoint of the deliverable period.

In one implementation, when there are a plurality of the set myocardial positions, the pulse stimulation control method according to this embodiment further includes the following steps:

For the sensing event LS in the local myocardial electrocardiogram corresponding to each myocardial position, steps S102 to S106 are executed one by one to timely and effectively deliver pulse stimulation to each myocardial position, ensuring the safety, effectiveness, and reliability of patient treatment.

Where the sensing time window is determined based on the first sensing time of the R-wave in the preset electrocardiogram and the second sensing time corresponding to the sensing event that occurs first.

For example, an illustration is provided by pre-setting a pulse stimulation electrode at each of three different myocardial positions (A, B, and C) in a patient, the set myocardial positions A, B, and C correspond to stimulation electrode pairs E1, E2, and E3 respectively, and the second sensing times of the corresponding sensing events are LS1, LS2, and LS3 respectively, wherein the occurrence times of LS1, LS2, and LS3 are sequential (i.e., sensing event LS1 occurs first, followed by the other sensing events in subsequent times). Where each electrode pair may be composed of: a unipolar electrode with a unipolar electrode, a bipolar electrode with a bipolar electrode lead, and a combination of a unipolar electrode with a unipolar electrode and a bipolar electrode with a bipolar electrode lead. Additionally, other types of electrodes may be used. The specific types of electrode pairs and how they are combined may be determined or adjusted based on the requirements of the actual scenario.

Specifically, when acquiring, based on the electrode pair E1 at the set myocardium position A, the sensing event LS1 from the local myocardial electrocardiogram corresponding to the myocardial position, the second sensing time corresponding to the sensing event LS1 is acquired, and whether the second sensing time falls within the sensing time window corresponding to the R-wave in the preset electrocardiogram is determined; if the second sensing time does not fall within the window, it is determined that the sensing event LS1 is not the R-wave signal corresponding to the R-wave in the preset electrocardiogram, but rather other interference signals such as T-waves, and thus, the pulse stimulation is prevented from being delivered to the stimulation electrode pair E1 at the set myocardial position A; if the second sensing time falls within the window, it is determined that the sensing event LS1 is the local myocardial R-wave signal corresponding to the R-wave in the preset electrocardiogram, and then the pulse delivery time corresponding to the stimulation electrode corresponding to the set myocardial position A is timely and accurately calculated at the second sensing time corresponding to the sensing event LS 1; next, it is determined whether the pulse delivery time falls within the deliverable pulse time window corresponding to the R-wave in the preset electrocardiogram, and if it does, the pulse stimulation is controlled to be delivered to the stimulation electrode at the set myocardial position A at the pulse delivery time, otherwise, it is determined that no pulse stimulation will be delivered to the stimulation electrode at the set myocardial position A, thus completing one pulse stimulation control for the set myocardial position A.

Similarly, the pulse stimulation control process for the set myocardial positions B and C is analogous to the pulse stimulation control process for the set myocardial position A, and therefore, the details are not described herein.

It should be noted that the pulse stimulation control processes for different set myocardial positions are independent of each other and do not cause mutual interference or influence. For example, during the pulse stimulation control process at the set myocardial position A, or after the completed pulse stimulation control at the set myocardial position A, as long as a sensing event LS2 appears in the local myocardial electrocardiogram corresponding to the set myocardial position B, the aforementioned pulse stimulation control process can be independently executed, and the pulse stimulation control for all set myocardial positions will ultimately completed in an orderly and efficient manner, thereby effectively guaranteeing the safety and reliability of pulse stimulation for patients.

In one implementation, when the sensing times corresponding to the sensing events of a plurality of local myocardial electrocardiograms occur very close together (i.e., within a small time span), the pulse stimulation control method according to this embodiment further includes the following steps:
acquiring the second sensing time corresponding to the sensing event LS in the local myocardial electrocardiogram corresponding to each local myocardial electrocardiogram to identify the sensing event which occurs earliest as the first sensing event LS1; acquiring the second sensing time corresponding to the first sensing event LS1, determining whether the first sensing event LS1 falls within the sensing time window corresponding to the R-wave in the preset electrocardiogram, and if the sensing event falls within the window, determining the sensing event as the local myocardial R-wave signal corresponding to the R-wave in the preset electrocardiogram; and
for the sensing events in the local myocardial electrocardiograms corresponding to the remaining myocardial positions, determining whether the second sensing times corresponding to these sensing events fall within the aforementioned sensing time window, and if yes, directly determining that the sensing events in the local myocardial electrocardiograms corresponding to the remaining myocardial positions are also R-wave signals corresponding to the R-wave in the preset electrocardiogram once the first sensing event is determined as the R-wave signal corresponding to the R-wave in the preset electrocardiogram. In this case, there is no need for individual judgment and analysis of the sensing events in the local myocardial electrocardiograms corresponding to the remaining myocardial positions, achieving accurate judgment while greatly simplifying the data analysis process, effectively reducing data processing time, and lowering the computational power requirements of devices. This further ensures the timeliness, accuracy, and effectiveness of the pulse stimulation control for patients.

It should be noted that the specific method used to determine whether the sensing events in multiple local myocardial electrocardiograms are R-waves may be selected based on the requirements of the actual scenario. A single execution scheme may be selected or multiple execution schemes may be combined to meet the higher demands of the cardiac electrical ventricular conduction scenario. This greatly enhances the practicality of pulse stimulation control and significantly improves the safety and effectiveness of patient treatment.

In addition, when there are a plurality of set myocardial positions, the pulse stimulation control method according to this embodiment further includes:
(1) presetting set sensing parameters corresponding to the R-waves at different set myocardial positions,
   wherein the set sensing parameter includes the set sensing time and/or the set sensing occurrence sequence;
(2) setting the sensing events in the other remaining local myocardial electrocardiograms all as R-waves if the sensing event in the local myocardial electrocardiogram that occurs first is an R-wave; or,
(3) setting the sensing events in the other remaining local myocardial electrocardiograms all as R-waves if the sensing event in the local myocardial electrocardiogram that occurs last is an R-wave.

Moreover, the above settings may be adjusted or additional settings may be added based on the requirements of the actual scenario.

The following specifies the implementation principle of determining whether a sensing event in the local myocardial electrocardiogram is an R-wave:
Determining whether the sensing event LS is an R-wave involves two stages: the set-up/pre-set period and the operational period.

For a pulse stimulation system with an electrode for a single myocardial point, this pulse stimulation system will have a preset electrocardiogram (such as ECG) and an electrode lead connected to the myocardium of the ventricle to sense the R-wave, and the system is used to deliver pulse stimulation to the myocardium. During the set-up period, the following parameters need to be measured:

### Set-up Period:

(11) ECG Sense (GS) = The time when the ECG (R-wave) sense occurs (global sense),
wherein the ventricular electrical activity of the R-wave in the preset electrocardiogram (ECG) represents the overall ventricular electrical activity, and R-wave sensing reflects a relatively early time of the ventricular electrical activity.

(12) EGM Sense (LS) = The time when the EGM (R-wave) sense occurs (local sense),
wherein LS is the local electrocardiogram sensing event that sensed first after the reference time starting point corresponding to GS (GS-X, X=60 ms, X can be programmatically controlled within a range of, but not limited to, 30 ms to 200 ms).

(13) GLSD=LS-GS, which is the time delay between GS and LS.

(14) LPD = The time delay between LS and the pulse stimulation;
(15) GPD = GLSD + LPD, which is the time delay between GS and the pulse stimulation.

(16) GVT is the sensing time window of the corresponding GS (programmable and adjustable); LSVT is the sensing time window of the corresponding LS;
specifically, the start time of GVT is GVT-s, and the duration of the time window is B;
a1) GVT-s=GS-A, GLSD>0 (i.e., LS occurs after GS);
   GVT-s=GS+GLSD-A, GLSD≤0 (i.e., LS occurs at the same time as or before GS),
   wherein the default value of A is 20 ms, and A may be programmed and adjusted (including 0 ms);
   in this case, GVT-s is acquired via the corresponding time parameters of GS and LS.
b1) For sinus rhythm, B ranges from 30 ms to 130 ms (programmable), but is not limited to this range;
c1) For ventricular ectopic excitation, such as PVCs or ventricular paced beats, B ranges from 30 ms to 250 ms (programmable) but is not limited to this range.

LSVT-s=LS-A; LSVT-_{B}=B-(LSVT-s - GVT-s).

It should be noted that LSVT is only used when there are electrodes in a plurality of local myocardial positions; additionally, LS is LS1, i.e., corresponds to the sensing event which occurs earliest among all myocardial sites.

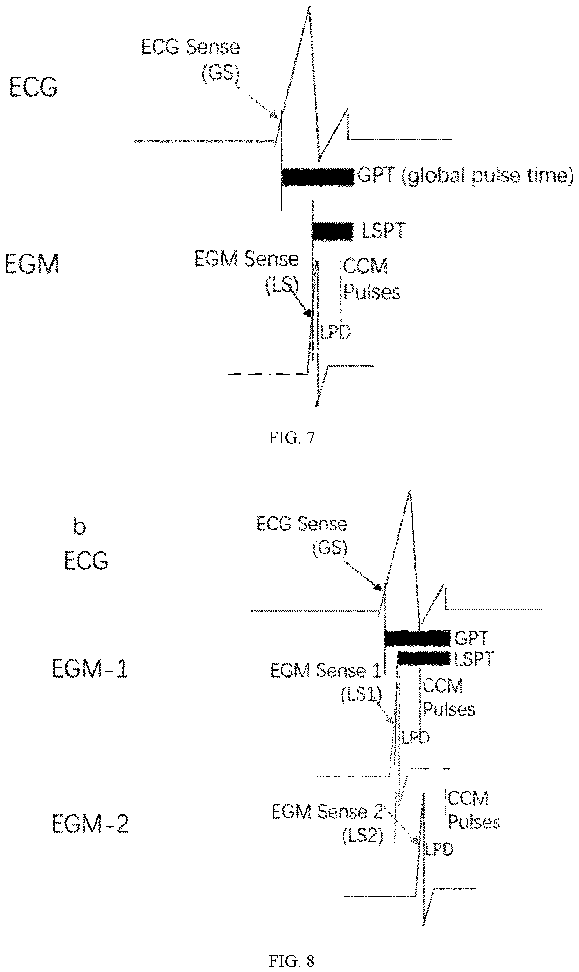

(17) As shown in FIG. 7, GPT is the deliverable pulse time window of the corresponding GS; LSPT is the pulse delivery event window of the corresponding LS (programmable and adjustable).

Specifically, the start time of GPT is GPT-s, and GPT-s =GVT-s; the window duration specifically includes, but is not limited to, the range of 20 ms to 200 ms (programmable), with a default of 130 ms.

The start time of LSPT is LSPT-s, and LSPT-s =LSVT-s, and the window duration of LSPT is the window duration of GPT minus GLSD.

It should be noted that the above description includes the scenario where GVT-s corresponds to far-field sensing GS in the preset electrocardiogram and LSVT-s corresponds to near-field sensing LS in the local myocardial electrocardiogram (i.e., A = 0 ms).

It should be noted that the above parameters are averaged over several cardiac cycles (e.g., 6 cardiac cycles, which is programmable and may also be a different number of cycles); additionally, the set-up period should be conducted separately during sinus electrical activity, ventricular ectopic activity, ventricular pacing, and the like.

As shown in FIG. 7, for a single electrode lead stimulation system, LSPT is the deliverable pulse time window of the corresponding LS, the window starts at LSVT-s, and the window duration is the window duration of GPT (B) minus GLSD. Both serve as the deliverable pulse time window using LS as the trigger point (reference point) for pulse stimulation delivery.

It should be noted that LSPT is only used when there are electrodes in a plurality of local myocardial positions; additionally, LS is LS1, i.e., corresponds to the sensing event which occurs earliest among all myocardial sites. In this case, the starting point of GPT also is acquired via the corresponding time parameters of LS.

In addition, in this embodiment, the time windows such as GVT, GPT, LSVT, and LSPT are all described by two parameters, i.e., the corresponding window starting point and the window duration.

### Operational Period:

(18) LS as a real-time local R-wave sensing needs to meet the following conditions:
a2) There are two sensing events: the preset ECG (GS) and the local EGM (LS),
   wherein LS is the second sensing time of the local electrocardiogram sensing event that is sensed first after the reference time starting point corresponding to GS (GS-X, X=60 ms, X can be programmatically controlled within a range of, but not limited to, 30 ms to 200 ms);
b2) LS falls within GVT (i.e., the sensing time window).

(19) The criteria for determining whether the pulse stimulation time is appropriate:
a3) The pulse stimulation time falls within GPT (the deliverable pulse time window).

Where GVT and GPT are derived from the corresponding ventricular electrical activities acquired during the set-up period, such as sinus electrical activity, ventricular ectopic activity, or ventricular pacing.

For a pulse stimulation system with a plurality of electrodes, this pulse stimulation system will have a preset electrocardiogram and a plurality of leads connected to multiple myocardial sites of the ventricles to sense the R-wave, and the system is used to deliver pulse stimulation to the local myocardium. During the set-up period, the following parameters need to be measured:

### Set-up Period:

(21) Similar to the above (11) to (19), measure and acquire all parameters corresponding to each stimulation electrode pair (as some programmatically controlled numbers together with the measured numbers constitute all parameters).

(22) Pre-form templates corresponding to LSn (local R-wave sensing), which include the time of LSn (n=1, 2, 3, ...) relative to LS1, and information such as the sequence of the occurrence of each electrode sensing.

### Operational Period:

(23) Acquire GS and LSn in real time during the operation.

(24) Apply the rules from step (18) above to determine whether each LSn is a true R-wave.

Alternatively, if LS1 is confirmed as a true R-wave in step (18), then LSn (n=2, 3, ...) is assumed to be a true R-wave (if LSn occurs within the GVT window).

Alternatively, if LS1 is confirmed as a true R-wave in step (18) and LSn (n=2, 3, ...) conforms to the preset template, then LSn (n=2, 3, ...) is separately confirmed as a true R sensing.

Alternatively, if LS1 is used as a reference, the sensing time window will be LSVT, and it is determined whether LSn (n=2, 3, ...) is within the LSVT time window.

As shown in FIG. 8, for a multi-electrode lead stimulation system during the setup period: if LS1 is used as a reference, the deliverable pulse time window is LSPT.

Operational period: after confirming each LS as R-wave sensing, the corresponding pulse delivery time should be within the GPT window; if LS1 is used as the trigger point (reference point) for the deliverable pulse time window, then the delivery time of LSn (n>1) is within the LSPT window.

It should be noted that for single-electrode and multi-electrode pulse stimulation systems, the pulse stimulation delivery time relative to each myocardial position is after the local myocardial sensing time, for example, after 40 ms. GPT or LSPT represents new requirements for pulse stimulation delivery time, especially in cases where there is pulse stimulation delivery for multiple stimulation sites. The delivery time of each stimulation site needs to fall within the time window of GPT or LSPT, that is, within the deliverable period of the entire ventricle of the same heartbeat (ventricular excitation), not just the deliverable period of local ventricular muscle at the electrode site.

GVT/LSVT, as a "global ventricular" or "far-field ventricular" R-wave sensing time window, is used to determine if LS is R-wave sensing of local ventricular depolarization corresponding to GS (i.e., corresponding to the "global ventricular" or "far-field" R-wave sensing of ventricular depolarization). GPT/LSPT, as the deliverable pulse time window (serving as a safety zone for stimulation delivery, corresponding to the deliverable period of the ventricular muscle as a whole ("global ventricle" or "far-field ventricle")), is used to determine if the pulse stimulation delivery time corresponding to LS is safe. GPT and GVT are independent parameters that can be separately controlled by a program to meet actual parameter configuration requirements. Moreover, to reduce the complexity of programming control, doctors may select the same numerical values for both parameters (when appropriate). Alternatively, the system may directly assign the same numerical value to both parameters in advance, but the functionality of programmatically controlling the two parameters separately must be maintained. The two steps may be used together or separately.

In addition, besides the aforementioned method of first setting a set-up period and then performing an operational period (i.e., the operational period relies on the numbers obtained during the set-up period for specific execution), the parameters may also be directly obtained and used during each electrical activity of ventricular muscle (each cardiac cycle) in the operational period, without relying on the set-up period. This greatly enhances the flexibility and efficiency of the pulse stimulation control process.

In this embodiment, pulse stimulation is delivered only after the R-wave, allowing for timely analysis and processing of the sensing event in the local myocardial electrocardiogram of the myocardium. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, thereby preventing unnecessary pain or even safety hazards to patients. This ensures patient safety and enhances the reliability of pulse stimulation control; additionally, it ensures that pulse stimulation is delivered timely when the sensing event is confirmed as an R-wave, that is, pulse stimulation is delivered only under correct conditions.

In addition, pulse stimulation is only delivered during the ventricular deliverable period: By confirming that the pulse stimulation delivery time falls within the pulse delivery window corresponding to the overall ventricular electrical activity (R-wave) in the surface electrocardiogram or the *in vivo* far-field myocardial electrocardiogram, the timeliness, safety, and effectiveness of pulse stimulation to the patient's heart are guaranteed. Where for the safety of the pulse stimulation transmission time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of pulse stimulation for the patient.

### Embodiment 3

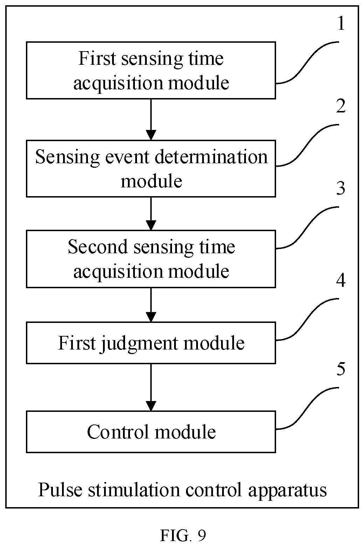

As shown in FIG. 9, the pulse stimulation control apparatus according to this embodiment includes:
a first sensing time acquisition module 1, configured to acquire the first sensing time of the R-wave in a preset electrocardiogram,
wherein the preset electrocardiogram includes but is not limited to the surface electrocardiogram (ECG) and the *in vivo* far-field myocardial electrocardiogram (Far-Field Electrogram); the first sensing time is also referred to as GS;
a sensing event determination module 2, configured to determine the sensing event in the local myocardial electrocardiogram corresponding to the set myocardial position based on the first sensing time;
a second sensing time acquisition module 3, configured to acquire the second sensing time corresponding to the sensing event,
wherein the second sensing time corresponding to the sensing event in the local myocardial electrocardiogram is also referred to as LS;
the sensing event in the local myocardial electrocardiogram is a sensing event corresponding to the R-wave in the preset electrocardiogram;
a first judgment module 4, configured to determine that the sensing event is not the R-wave signal corresponding to the R-wave in the preset electrocardiogram if the second sensing time does not meet the first preset condition and to call the control module 5; and
a control module 5, configured to not deliver the pulse stimulation to the stimulation electrode at the set myocardial position,
wherein the first preset condition is determined based on the first sensing time of the R-wave in the preset electrocardiogram and the second sensing time corresponding to the sensing event.

In one implementation, the first judgment module 4 is further configured to acquire the sensing time window corresponding to the R-wave in the preset electrocardiogram; the first judgment module 4 is further configured to determine that the sensing event is not the R-wave signal corresponding to the R-wave in the preset electrocardiogram if the second sensing time does not fall within the sensing time window.

Where the method for acquiring the sensing time window includes but is not limited to: obtaining the sensing time window based on a preset sensing circuit or directly receiving the sensing time window through an external device input.

Non-R-wave signals corresponding to the sensing event LS in the local myocardial electrocardiogram include T-wave signals or other interference signals. Specifically, pulse stimulation is CCM stimulation; allowing pulse stimulation to be implemented under different ventricular electrical activities expands the applicability of pulse stimulation, significantly enhancing the overall effectiveness of pulse stimulation control.

CCM stimulation occurs during the following ventricular electrical activities: sinus beats, ventricular beats resulting from atrial conduction, ventricular beats originating from the ventricles (ventricular ectopic excitations), ventricular paced beats, etc.

Confirming that the local sensing event is an R-wave better ensures that CCM stimulation is triggered by the local R-wave instead of other signals (such as T-waves, myoelectricity, or other non-myocardial depolarization electrical activities), effectively eliminating the occurrence of false triggers. Through the design of this critical step, the safety and effectiveness of pulse stimulation can be effectively improved.

It should be noted that the implementation principle of the pulse stimulation control apparatus in this embodiment is similar to that of the pulse stimulation control method in Embodiment 1, and therefore, the details are not described herein.

In this embodiment, the sensing event in the local myocardial electrocardiogram for each heartbeat can be analyzed and processed timely. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, and preventing unnecessary pain or even safety hazards to patients. This ensures patient safety, enhances the reliability of pulse stimulation control, and guarantees the safety, effectiveness, and therapeutic effects of pulse stimulation for patients.

### Embodiment 4

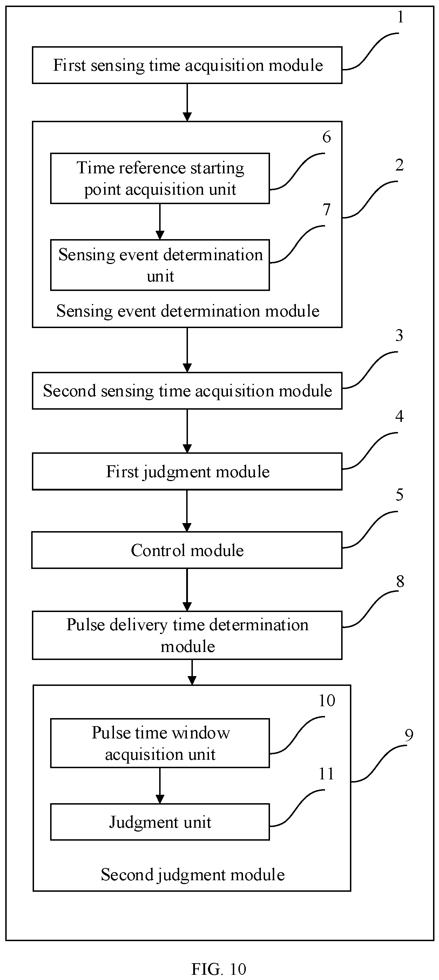

As shown in FIG. 10, the pulse stimulation control system according to this embodiment is a further improvement of Embodiment 3, specifically:
In one implementation, the sensing event determination module 2 according to this embodiment includes:
a time reference starting point acquisition unit 6, configured to take the first sensing time as the first time point and take the time point from corresponding first set duration before the first time point as the time reference starting point; and
a sensing event determination unit 7, configured to acquire the sensing event in the local myocardial electrocardiogram corresponding to the set myocardial position based on the time reference starting point.

In one implementation, the first judgment module 4 is further configured to determine the sensing event as an R-wave if the second sensing time falls within the sensing time window.

In one implementation, the pulse stimulation control system according to this embodiment further includes:
a pulse delivery time determination module 8, configured to determine, after the sensing event is determined as an R-wave, the pulse delivery time corresponding to the stimulation electrode at the set myocardial position based on the second sensing time of the R-wave in the local myocardial electrocardiogram.

It should be noted that in this embodiment, the method of acquiring sensing events as R-waves may be directly obtained through transmission by external devices, or determined based on judgments such as whether it falls within the sensing time window.

Specifically, the pulse delivery time determination module 8 is configured to calculate the pulse delivery time corresponding to the stimulation electrode at the set myocardial position based on the second sensing time of the R-wave in the local myocardial electrocardiogram and the preset duration.

Alternatively, the pulse delivery time determination module 8 is configured to acquire the first sensing time corresponding to the R-wave in the preset electrocardiogram; calculate the time difference between the first sensing time and the second sensing time at the set myocardial position; and calculate, using the first sensing time as the reference zero point, the pulse delivery time corresponding to the set myocardial position based on the time difference and the preset duration.

A second judgment module 9 is configured to determine whether the pulse delivery time meets the second preset condition; if yes, the judging module is called to determine to deliver pulse stimulation to the stimulation electrode at the set myocardial position; otherwise, the judging module is called to not deliver pulse stimulation to the stimulation electrode at the set myocardial position.

The determination of the sensing event of which the local myocardial sensing time is within the sensing time window corresponding to the R-wave in the preset electrocardiogram ensures that the sensing event belongs to the R-wave signal of the local myocardium corresponding to the R-wave in the preset electrocardiogram. This guarantees the timely delivery of pulse stimulation only when the event is determined as an R-wave, ensuring the timeliness, safety, and effectiveness of pulse stimulation for the patient's heart.

In one implementation, the second judgment module 9 according to this embodiment includes:
a pulse time window acquisition unit 10, configured to acquire the deliverable pulse time window corresponding to the R-wave in the preset electrocardiogram,
wherein the deliverable pulse time window is set to correspond with the sensing time window; and
a judgment unit 11, configured to call the control module 5 to control the pulse stimulation to be delivered to the stimulation electrode at the set myocardial position in the pulse delivery time if the pulse delivery time falls within the deliverable pulse time window, and
further configured to call the control module 5 to not deliver the pulse stimulation to the stimulation electrode at the set myocardial position if the pulse delivery time does not fall within the deliverable pulse time window.

In one implementation, when there are a plurality of the set myocardial positions, the control module 5 in this embodiment is configured to control the first judgment module 4 to determine whether the corresponding second sensing time falls within the sensing time window for each of the second sensing times of the sensing events occurring in each of the local myocardial electrocardiograms in sequence.

Where the sensing time window is determined based on the first sensing time of the R-wave in the preset electrocardiogram and the second sensing time corresponding to the sensing event that occurs first.

In one implementation, when there are a plurality of the set myocardial positions, the control module 5 in this embodiment is configured to preset set sensing parameters corresponding to the R-wave at different set myocardial positions,
wherein the set sensing parameter includes the set sensing time and/or the set sensing occurrence sequence;
   or,
set the sensing events in the other remaining local myocardial electrocardiograms all as R-waves if the sensing event in the local myocardial electrocardiogram that occurs first is an R-wave;
   or,
set the sensing events in the other remaining local myocardial electrocardiograms all as R-waves if the sensing event in the local myocardial electrocardiogram that occurs last is an R-wave.

It should be noted that the working principle of the pulse stimulation control apparatus in this embodiment is similar to the implementation principle of pulse stimulation control in Embodiment 2, and therefore, the details are not described herein.

In this embodiment, pulse stimulation is delivered only after the R-wave, allowing for timely analysis and processing of the sensing event in the local myocardial electrocardiogram of the myocardium. False sensing events are automatically and accurately identified and excluded, and they are also determined as interference signals such as the T-wave instead of the R-wave signal corresponding to the R-wave in the preset electrocardiogram. In this case, pulse stimulation is prevented from being delivered to the corresponding myocardial position, ensuring that pulse stimulation is not delivered under incorrect conditions, effectively avoiding the risk of inducing VT or VF, thereby preventing unnecessary pain or even safety hazards to patients. This ensures patient safety and enhances the reliability of pulse stimulation control; additionally, it ensures that pulse stimulation is delivered timely when the sensing event is confirmed as an R-wave, that is, pulse stimulation is delivered only under correct conditions.

In addition, pulse stimulation is only delivered during the ventricular deliverable period: By confirming that the pulse stimulation delivery time falls within the pulse delivery window corresponding to the overall ventricular electrical activity (R-wave) in the surface electrocardiogram or the *in vivo* far-field myocardial electrocardiogram, the timeliness, safety, and effectiveness of pulse stimulation to the patient's heart are guaranteed. Where for the safety of the pulse stimulation transmission time, using the surface electrocardiogram or the far-field myocardial electrocardiogram to obtain the deliverable period information of ventricular electrical activity also represents a positive improvement in pulse stimulation technology, further ensuring the safety, effectiveness, and therapeutic effect of pulse stimulation for the patient.

### Embodiment 5

The medical system ECS in this embodiment includes the pulse stimulation control apparatus in Embodiments 3 or 4.

Global myocardial depolarization (R-wave and corresponding GS) may originate from, but is not limited to, a surface electrocardiogram (from surface electrocardiogram electrodes attached to the skin), or a far-field myocardial electrocardiogram (FF-EGM, from electrodes near the heart or electrodes distant from the heart but located subcutaneously within the body in tissues or organs). This allows the ECS system to be used in various devices or equipment that provide cardiac therapy, such as AEDs, MCS (Mechanical Circulatory Support), subQ-ICDs, and implantable CRM (Cardiac Rhythm Management) devices, among others. Additionally, this enables the ECS to serve not only as an acute support device (with surface electrocardiogram ECG as one of the input signals) but also as a chronic heart failure treatment device, although surface ECG cannot always be used as an input for the device. In this way, the ECS system can provide treatment to more patients in more scenarios, thereby benefiting more patients. The medical system in this embodiment allows pulse stimulation to be delivered during sinus rhythm, ventricular rhythm (beats) conducted from the atrium, or other ventricular ectopic rhythms (beats). This represents a breakthrough or improvement over the current limitation where pulse stimulation cannot be delivered during heartbeats under a non-normal cardiac conduction system, such as sinus beats, ventricular beats resulting from atrial conduction, ventricular beats originating from the ventricles (ventricular ectopic excitations), and ventricular paced beats. This will provide more opportunities for cardiac support, especially in situations where the patient may need such support the most.

In this embodiment, the medical system integrates the aforementioned pulse stimulation control apparatus, which can ensure that pulse stimulation is only delivered under correct conditions and not under incorrect conditions. Moreover, the medical system delivers pulse stimulation within the deliverable period corresponding to the R-wave in surface electrocardiograms and *in vivo* far-field myocardial electrocardiograms, ensuring the timeliness and effectiveness of the pulse stimulation to the patient's heart. This guarantees the safety, effectiveness, and therapeutic efficacy of the pulse stimulation for the patient, thereby effectively enhancing the overall product performance of existing medical systems.

### Embodiment 6

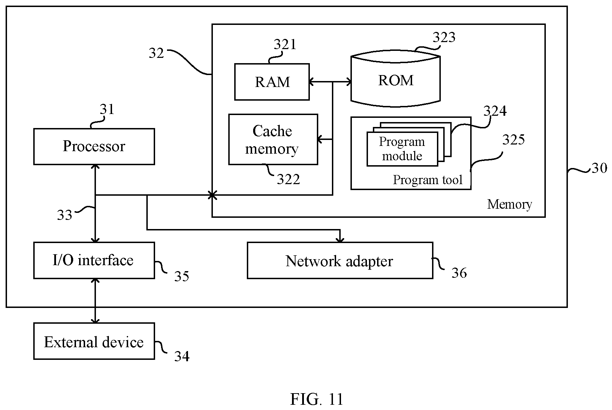

FIG. 11 is a structural schematic diagram of an electronic device provided according to Embodiment 6 of the present disclosure. The electronic device includes a memory, a processor, and a computer program stored on the memory and executable by the processor. The processor, when executing the program, implements the pulse stimulation control method according to Embodiment 1 or 2. The electronic device 30 shown in FIG. 11 is only an example, and should not bring any limitation to the functions and the use scope of the embodiments of the present disclosure.

As shown in FIG. 11, the electronic device 30 may take the form of a general-purpose computing device, such as a server device. Components of the electronic device 30 may include, but are not limited to: at least one processor 31, at least one memory 32, and a bus 33 connecting various system components (including the memory 32 and the processor 31).

The bus 33 includes a data bus, an address bus, and a control bus.

The memory 32 may include a volatile memory, such as a random access memory (RAM) 321 and/or a cache memory 322, and may further include a read-only memory (ROM) 323.

The memory 32 may further include programs/utilities 325 with a set of (at least one) program modules 324, such program modules 324 including but not limited to: an operating system, one or more application programs, other program modules, and program data. Each of these examples or some combination thereof may include the implementation of a network environment.

The processor 31 executes various functional applications and performs data processing, such as the pulse stimulation control method according to Embodiment 1 or 2 of the present disclosure, by executing the computer program stored in the memory 32.

The electronic device 30 may also communicate with one or more external devices 34 (such as a keyboard and a pointing device). Such communication may be implemented through an input/output (I/O) interface 35. In addition, a model-generating device 30 may also communicate with one or more networks (such as a local area network (LAN), a wide area network (WAN), and/or a public network, such as the Internet) via a network adapter 36. As shown in FIG. 11, the network adapter 36 communicates with other modules of the model-generating device 30 via the bus 33. It should be understood that although not shown in the figures, other hardware and/or software modules may be used in conjunction with the model-generating device 30, including, but not limited to: microcode, device drivers, redundant processors, external disk drive arrays, RAID (disk array) systems, tape drives, data backup storage systems, and the like.

It should be noted that although several units/modules or sub-units/modules of the electronic device are mentioned in the above detailed description, such a division is merely exemplary but not mandatory. Indeed, the features and functions of two or more of the units/modules described above may be embodied in one unit/module according to embodiments of the present disclosure. Conversely, the features and functions of one unit/module described above may be further divided to be embodied by a plurality of units/modules.

### Embodiment 7

This embodiment provides a computer-readable storage medium storing a computer program. The program, when executed by a processor, causes the processor to implement the steps of the pulse stimulation control method in Embodiment 1 or 2.

Where the readable storage medium may be employed more specifically and may include, but is not limited to: a portable disk, a hard disk, a random access memory, a read-only memory, an erasable programmable read-only memory, an optical storage device, a magnetic storage device, or any suitable combination of the foregoing.

In a possible implementation, the present disclosure can also be implemented in the form of a program product comprising program codes used for causing a terminal device to implement the steps of the pulse stimulation control method in Embodiment 1 or 2 when the program product is executed on the terminal device.

Where the program codes used for implementing the present disclosure are written in any combination of one or more programming languages, and the program codes may be executed entirely on a user device, partly on the user device, as a stand-alone software package, partly on the user device and partly on a remote device, or entirely on the remote device.

Although specific embodiments of the present disclosure have been described above, it should be understood by those skilled in the art that these embodiments are merely illustrative and that the protection scope of the present disclosure is defined by the appended claims. Various changes or modifications may be made to these embodiments by those skilled in the art without departing from the principle and spirit of the present disclosure, and such changes and modifications shall fall within the protection scope of the present disclosure.

## Claims

1. A pulse stimulation control method, **characterized by** comprising:
acquiring a first sensing time of an R-wave in a preset electrocardiogram;
determining a sensing event in a local myocardial electrocardiogram corresponding to a set myocardial position based on the first sensing time, and acquiring a second sensing time corresponding to the sensing event; and
determining that the sensing event is not an R-wave signal corresponding to the R-wave in the preset electrocardiogram if the second sensing time does not meet a first preset condition, and then not delivering pulse stimulation to a stimulation electrode at the set myocardial position,
wherein the first preset condition is determined based on the first sensing time of the R-wave in the preset electrocardiogram and the second sensing time corresponding to the sensing event.

2. The pulse stimulation control method according to claim 1, **characterized in that** when the second sensing time does not meet the first preset condition, determining that the sensing event is not the R-wave signal corresponding to the R-wave in the preset electrocardiogram, and then not delivering the pulse stimulation to the stimulation electrode at the set myocardial position, comprises:
acquiring a sensing time window corresponding to the R-wave in the preset electrocardiogram; and
when the second sensing time does not fall within the sensing time window, determining that the sensing event is not the R-wave signal corresponding to the R-wave in the preset electrocardiogram, and then not delivering the pulse stimulation to the stimulation electrode at the set myocardial position.

3. The pulse stimulation control method according to claim 1 or 2, **characterized in that** determining the sensing event in the local myocardial electrocardiogram corresponding to the set myocardial position based on the first sensing time comprises:
taking the first sensing time as a first time point, and taking a time point from corresponding first set duration before the first time point as a time reference starting point; and
acquiring the sensing event in the local myocardial electrocardiogram corresponding to the set myocardial position based on the time reference starting point.

4. The pulse stimulation control method according to at least one of claims 1 to 3, **characterized by** further comprising:
determining the sensing event as an R-wave if the second sensing time falls within the sensing time window.

5. The pulse stimulation control method according to at least one of claims 1 to 4, **characterized by** further comprising:
determining, after the sensing event is determined as the R-wave, a pulse delivery time corresponding to the stimulation electrode at the set myocardial position based on the second sensing time of the R-wave in the local myocardial electrocardiogram; and
determining whether the pulse delivery time meets a second preset condition, and if yes, determining to deliver the pulse stimulation to the stimulation electrode at the set myocardial position; otherwise, not delivering the pulse stimulation to the stimulation electrode at the set myocardial position.

6. The pulse stimulation control method according to claim 5, **characterized in that** determining whether the pulse delivery time meets the second preset condition, and if yes, determining to deliver the pulse stimulation to the stimulation electrode at the set myocardial position; otherwise, not delivering the pulse stimulation to the stimulation electrode at the set myocardial position, comprises:
acquiring a deliverable pulse time window corresponding to the R-wave in the preset electrocardiogram,
wherein the deliverable pulse time window is set to correspond with the sensing time window;
controlling the pulse stimulation to be delivered to the stimulation electrode at the set myocardial position in the pulse delivery time if the pulse delivery time falls within the deliverable pulse time window; and
not delivering the pulse stimulation to the stimulation electrode at the set myocardial position if the pulse delivery time does not fall within the deliverable pulse time window.

7. The pulse stimulation control method according to at least one of claims 2 to 6, **characterized in that** when there are a plurality of the set myocardial positions, the method further comprises:
determining whether the corresponding second sensing time falls within the sensing time window for each of the second sensing times of the sensing events occurring in each of the local myocardial electrocardiograms in sequence,
wherein the sensing time window is determined based on the first sensing time of the R-wave in the preset electrocardiogram and the second sensing time corresponding to the sensing event that occurs first.

8. The pulse stimulation control method according to at least one of claims 1 to 7, **characterized in that** when there are a plurality of the set myocardial positions, the method further comprises:
presetting set sensing parameters corresponding to sensing times at different set myocardial positions,
wherein the set sensing parameter comprises a set sensing time and/or a set sensing occurrence sequence; or,
setting sensing events in the other remaining local myocardial electrocardiograms all as R-waves if the sensing event in the local myocardial electrocardiogram that occurs first is an R-wave; or,
setting the sensing events in the other remaining local myocardial electrocardiograms all as R-waves if the sensing event in the local myocardial electrocardiogram that occurs last is an R-wave.

9. The pulse stimulation control method according to claims 6 or 7, **characterized in that** the preset electrocardiogram comprises a surface electrocardiogram and/or an *in vivo* far-field myocardial electrocardiogram;
and/or,
the deliverable pulse time window and the sensing time window are determined based on the preset electrocardiogram.

10. The pulse stimulation control method according to claim 5, **characterized in that** determining the pulse delivery time corresponding to the stimulation electrode at the set myocardial position based on the second sensing time of the R-wave in the local myocardial electrocardiogram comprises:
calculating the pulse delivery time corresponding to the stimulation electrode at the set myocardial position based on the second sensing time of the R-wave in the local myocardial electrocardiogram and a preset duration;
or,
calculating a time difference between the first sensing time corresponding to the R-wave in the preset electrocardiogram and the second sensing time of the set myocardial position; and
using the first sensing time as a reference zero point, calculating the pulse delivery time corresponding to the set myocardial position based on the time difference and the preset duration.

11. The pulse stimulation control method according to at least one of claims 1 to 10, **characterized in that** the pulse stimulation is CCM stimulation,
wherein the CCM stimulation may be delivered during at least one of the following ventricular electrical activities:
sinus beats, ventricular beats resulting from atrial conduction, ventricular beats originating from ventricles, and ventricular paced beats.

12. A pulse stimulation control apparatus, **characterized by** comprising:
a first sensing time acquisition module, configured to acquire a first sensing time of an R-wave in a preset electrocardiogram;
a sensing event determination module, configured to determine a sensing event in a local myocardial electrocardiogram corresponding to a set myocardial position based on the first sensing time;
a second sensing time acquisition module, configured to acquire a second sensing time corresponding to the sensing event; and
a first judgment module, configured to determine that the sensing event is not an R-wave signal corresponding to the R-wave in the preset electrocardiogram if the second sensing time does not meet a first preset condition, and then not deliver pulse stimulation to a stimulation electrode at the set myocardial position,
wherein the first preset condition is determined based on the first sensing time of the R-wave in the preset electrocardiogram and the second sensing time corresponding to the sensing event.

13. A medical system, **characterized by** comprising the pulse stimulation control apparatus according to claim 12.

14. An electronic device, comprising a memory, a processor, and a computer program stored on the memory and executable by the processor, **characterized in that** the processor, when executing the computer program, implements the pulse stimulation control method according to any one of claims 1 to 11.

15. A computer-readable storage medium storing a computer program, **characterized in that** the computer program, when executed by a processor, causes the processor to implement the pulse stimulation control method according to any one of claims 1 to 11.
